(19) **Europäisches Patentamt**

**European Patent Office**

**Office européen des brevets**

(11) **EP 1 037 166 A1**

(12) **EUROPÄISCHE PATENTANMELDUNG**

(43) Veröffentlichungstag:
20.09.2000 Patentblatt 2000/38

(51) Int. Cl.⁷: **G06T 5/00**

(21) Anmeldenummer: 00200798.7

(22) Anmeldetag: 07.03.2000

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(30) Priorität: **16.03.1999 DE 19911587**
**14.04.1999 DE 19916821**

(71) Anmelder:
• **Philips Corporate Intellectual Property GmbH**
**52064 Aachen (DE)**
Benannte Vertragsstaaten:
**DE**
• **Koninklijke Philips Electronics N.V.**
**5621 BA Eindhoven (NL)**
Benannte Vertragsstaaten:
**FR GB**

(72) Erfinder:
• **Wiemker, Rafael,**
**Philips Corp. Int. Prop. GmbH**
**52064 Aachen (DE)**
• **Dippel, Sabine,**
**Philips Corp. Int. Prop. GmbH**
**52064 Aachen (DE)**
• **Buzug, Thorsten,**
**Philips Corp. Int. Prop. GmbH**
**52064 Aachen (DE)**
• **Stahl, Martin,**
**Philips Corp. Int. Prop. GmbH**
**52064 Aachen (DE)**
• **Blaffert, Thomas,**
**Philips Corp. Int. Prop. GmbH**
**52064 Aachen (DE)**

(74) Vertreter:
**Volmer, Georg, Dipl.-Ing. et al**
**Philips Corporate Intellectual Property GmbH,**
**Habsburgerallee 11**
**52064 Aachen (DE)**

(54) **Verfahren zur Detektion von Konturen in einem Röntgenbild**

(57) Die vorliegende Erfindung betrifft ein Verfahren zum Detektieren von Konturen in einem Röntgenbild, wie sie beispielsweise von Blenden oder Implantaten erzeugt werden. Dabei wird aus dem Röntgenbild eine Anzahl geschlossener Pfade abgeleitet, von denen in Abhängigkeit vom Kontrast entlang dieser Pfade einer als Kontour ausgewählt wird. Die geschlossenen Pfade können aus einer Menge von Geraden-Kandidaten abgeleitet werden, auf denen Konturpunkt-Kandidaten liegen. Die Konturpunkt-Kandidaten ihrerseits können aus einem aus dem Röntgenbild abgeleiteten Hochpaß- oder Gradientenbild abgeleitet werden.

FIG. 1

**Beschreibung**

Die Erfindung betrifft ein Verfahren zur automatischen Detektion der Konturen von Strukturen mit hoher Röntgenstrahlenabsorption in einem Röntgenbild. Außerdem bezieht sich die Erfindung auf ein Röntgengerät zur Durchführung eines solchen Verfahrens.

In der radiologischen Praxis gibt es verschiedene Strukturen mit hoher Röntgenstrahlenabsorption, deren Konturen detektiert werden müssen, um eine optimale Verarbeitung des Röntgenbildes zu gewährleisten:

1. Die Konturen der Blenden, mit deren Hilfe das auf einen Patienten im Untersuchungsbereich treffende Röntgenstrahlenbündel begrenzt wird. Die genaue Kenntnis des räumlichen Verlaufs der Blendenkonturen in einem Röntgenbild läßt sich in vielfältiger Weise ausnutzen:

a) Der Dynamikbereich eines Monitors oder einer sogenannten Hardcopy-Einheit kann an den von der Blendenkontur umschlossenen Bildausschnitt automatisch so angepaßt werden, daß die Kontraste optimal wiedergegeben werden (autoranging).
b) Bei der Ausgabe eines Röntgenbildes kann der durch die Blenden abgedeckte Bereich schwarz oder farbig wiedergegeben werden. Ohne diese Maßnahme würden diese Bildpartien hell erscheinen, wodurch der Betrachter geblendet werden könnte.
c) Die Bilder können gedreht und — im Falle einer Schrägprojektion — entzerrt werden, so daß die Blendenkonturen auf dem wiedergegebenen Bild horizontal bzw. vertikal verlaufen. Außerdem können mehrere Bilder so nebeneinanderangeordnet werden, daß die zur Verfügung stehende Fläche auf einem Wiedergabemonitor bzw. auf einer Hardcopy optimal ausgenutzt wird.
d) Die auf das Röntgenbild anzuwendenden Bildverarbeitungsverfahren können auf den durch die Blendenkontur definierten Ausschnitt beschränkt werden. Dadurch läßt sich der Rechenaufwand für die Bildverarbeitung deutlich reduzieren.

2. Die Konturen von Implantaten — z.B. von einem künstlichen Hüftgelenk — in einem Röntgenbild. Solche Implantate haben eine Röntgenstrahlenabsorption, die stärker ist als die von Knochen aber schwächer als die der Röntgenstrahlenblende. Die Anwesenheit eines solchen Implantats in einem Röntgenbild ändert den mittleren Grauwert (Bildwert) und andere statistische Eigenschaften eines Grauwerthistogramms des Röntgenbildes. Dies kann dazu führen, daß die aus dem Röntgenbild abgeleitete automatische Einstellung einer Wiedergabeeinheit, auf der das Röntgenbild dargestellt wird, nicht optimal ist. Eine Verbesserung wäre hier möglich, wenn die Bereiche des Röntgenbildes, in denen das Implantat abgebildet wird, von der Histogrammanalyse ausgeschlossen würden, die die Grundlage für die Kontrasteinstellung des Wiedergabegerätes bildet.

Aus der US-PS 5 651 042 ist bereits ein Verfahren zur automatischen Detektion von Blendenkonturen in einem Röntgenbild bekannt, bei dem aus dem räumlichen Verlauf der den Pixeln des Röntgenbildes (oder eines daraus abgeleiteten Eingangsbildes) zugeordneten Bildwerte Konturpunkt-Kandidaten (d.h. Pixel, die auf der Kontur der Blende liegen können) bestimmt werden. Die Konturpunkt Kandidaten können aus einem aus dem Eingangsbild abgeleiteten Gradientenbild (oder einem Hochpaßbild) als diejenigen Punkte abgeleitet werden, für die der Gradient ein Maximum aufweist. Aus diesen Konturpunkt-Kandidaten werden dann mit Hilfe der linearen Regression Geraden-Kandidaten berechnet, die so angeordnet sind, daß die Konturpunkt-Kandidaten auf ihnen liegen bzw. in ihrer unmittelbaren Nachbarschaft. Die Geraden-Kandidaten können (müssen aber nicht) mit den Blendenkonturen zusammenfallen. In der Regel entstehen dabei mehr Geraden-Kandidaten.als die Blende Konturen hat. Unter diesen Geraden-Kandidaten werden diejenigen (maximal 4) ausgesucht, die am besten mit einem von 14 in einer Bibliothek gespeicherten Archetypen von Blendenkonturen übereinstimmen. Voraussetzung für dieses Verfahren ist, daß die Blendenkonturen parallel bzw. rechtwinklig zueinander verlaufen, und daß der von der Blendenkontur umschlossene Bereich zentral liegt.

Diese Voraussetzungen sind in der radiologischen Praxis nicht immer gegeben. Selbst wenn die Röntgenstrahlung mittels Blendenpaaren ausgeblendet werden, die zueinander parallel bzw. zueinander senkrecht verlaufen, sind im Falle einer Schrägprojektion die Blendenkonturen im Röntgenbild nicht mehr parallel oder senkrecht zueinander. Es kann auch vorkommen, daß ein Teil des Untersuchungsbereichs, auf den das Röntgenstrahlenbündel trifft, von einer Bleischürze oder dgl. abgedeckt wird, wobei nur in den seltensten Fällen deren Begrenzung im Röntgenbild parallel bzw. senkrecht zu den Blendenkanten verlaufen. Vielmehr kann sich im Röntgenbild eine Kontur ergeben, die nicht mehr mit einem Rechteck zusammenfällt.

Darüber hinaus können digitale Röntgenbildwandler, die das Röntgenbild in digitalisierbare elektrische Signale umsetzen, relativ große Abmessungen haben (z.B. 43 cm x 43 cm). Nimmt man mit einem solchen Röntgenbildwandler z.B. nur eine Hand oder einen Finger auf, dann wird das durch die Blenden begrenzte Röntgenbild in der Regel nicht in der Mitte liegen, sondern eher am Rande.

Auch bei dem aus der US-appln 08/715225 (PHQ 93-013) bekannten Verfahren ist Voraussetzung, daß die Blendenkanten möglichst parallel zu den Kanten des Eingangsbildes verlaufen und den Mittelpunkt umschließen.

Die gleiche Einschränkung gilt für das aus der US-PS 5 081 680 bekannte Verfahren, bei dem auf verschiedenen durch den Mittelpunkt verlaufenden Geraden die Punkte ermittelt werden, die einen maximalen Gradienten aufweisen. Sind auf einer Geraden mehr als zwei solche Punkte vorgesehen — was in einem normalen Röntgenbild mit Skelettstrukturen stets der Fall sein wird — müssen in einem gesonderten Ranking-Verfahren die zusätzlichen Punkte ausgeschlossen werden. Die verbleibenden Punkte werden als Punkte auf der Blendenkontur betrachtet, und aus diesen Blendenkonturpunkten wird mit Hilfe einer Hough-Transformation eine Anzahl von ein Polygon bildenden Geraden erzeugt, die die Blendenkontur darstellen sollen.

Aufgabe der vorliegenden Erfindung ist es ein Verfahren zur automatischen Detektion der Konturen von Strukturen mit hoher Röntgenstrahlenabsorption anzugeben, das zuverlässig arbeitet und nicht auf bestimmte Konturformen beschränkt ist. Diese Aufgabe wird erfindungsgemäß durch folgende Maßnahmen gelöst:

a) Ermittlung einer Anzahl von geschlossenen Pfaden in einem Röntgenbild oder einem daraus abgeleiteten Bild als Kontur-Kandidaten,
b) Auswahl eines geschlossenen Pfades — aus der Anzahl von geschlossenen Pfaden — als Kontur in Abhängigkeit von den Kontrasten entlang der geschlossenen Pfade.

Erfindungsgemäß werden also aus dem Röntgenbild eine Anzahl von geschlossenen Pfaden in dem Bild abgeleitet, von denen einer die gesuchte Kontur darstellt. Bei der Erfindung werden nun nicht einzelne Abschnitte der geschlossenen Pfade untersucht und verworfen bzw. bestätigt, und es werden auch nicht Pfade ausgewählt, die eine bestimmte Form haben. Es wird vielmehr von der Überlegung ausgegangen, daß sich entlang der Kontur besonders starke Kontraste zwischen den Bildwerten innerhalb und außerhalb des dadurch begrenzten Bildbereichs einstellen. Somit wird der geschlossene Pfad als Kontur ausgewählt, entlang dessen sich die stärksten Kontraste ergeben.

Anspruch 2 beschreibt ein für die Ermittlung einer Blendenkontur besonders geeignetes Verfahren. Unter den Konturpunkt-Kandidaten, die zunächst aus dem Eingangsbild abgeleitet werden, gibt es in der klinischen Praxis einen erheblichen Teil, der nicht auf einer Blendenkontur liegt. Bei der Ausgestaltung nach Anspruch 2 werden gleichwohl keine Versuche unternommen, solche Konturpunkt-Kandidaten von vornherein von der weiteren Verarbeitung auszuschließen. Sie dienen vielmehr dazu, Geraden-Kandidaten zu bestimmen, auf denen bzw. in deren unmittelbarer Nachbarschaft sich die Konturpunkt-Kandidaten befinden. Diese Geraden-Kandidaten können — zumindest stückweise — einen Teil der Blendenkontur bilden; sie können jedoch auch eine anatomischen Kontur im Röntgenbild zugeordnet sein.

Auch hier werden keine Versuche unternommen, solche Geraden-Kandidaten von der weiteren Verarbeitung auszuschließen. Es werden vielmehr geschlossene Pfade (cyclic paths) gebildet, also Polygone, deren Anfang mit ihrem Ende zusammenfällt. Aus den Abschnitten verschiedener Geraden-Kandidaten läßt sich eine Vielzahl von geschlossenen Pfaden zusammensetzen. Einer dieser geschlossenen Pfade stellt die Blendenkontur dar. Bei der Auswahl dieses einen Pfades als Blendenkontur wird von der Überlegung ausgegangen, daß sich entlang der Blendenkontur besonders starke Kontraste zwischen den Bildwerten innerhalb und außerhalb des dadurch begrenzten Bildbereichs einstellen.

Grundsätzlich könnten die geschlossenen Pfade auch anders als gemäß Anspruch 2 ermittelt werden, z.B. durch Ermittlung des räumlichen Verlaufs der Null-Durchgänge nach einer LoG-Filterung des Röntgenbildes. Die in Anspruch 2 beschriebene Ausgestaltung kann eine Blendenkontur jedoch auch dann noch zuverlässig ermitteln, wenn nur einzelne Abschnitte einer Blendenkante einen stärkeren Kontrast aufweisen.

Ein für die Ermittlung der Kontur eines Implantats geeignetes Verfahren ist in Anspruch 3 beschrieben. Dieses Verfahren arbeitet bei der Kontur-Detektion von Implantaten zuverlässig, weil diese Konturen in der Regel auf ihrem gesamten Umfang einen deutlichen Kontrast gegenüber ihrer Umgebung aufweisen. Die Bestimmung der geschlossenen Pfade benötigt bei dieser Ausgestaltung nur sehr wenig Rechenzeit.

Eine besonders zweckmäßige Ausgestaltung zur Ermittlung einer Blendenkontur ist in Anspruch 4 angegeben. Die Summierung der Kontraste entlang des geschlossenen Pfades kann dabei so erfolgen, wie in einem der Ansprüche 5 oder 6 angegeben. Es läßt sich zeigen, daß die in den Ansprüchen 5 und 6 angegebenen Formeln mathematisch identisch sind und sich ineinander überführen lassen.

In der Praxis gibt es eine Vielzahl von Kombinationen aus Stücken der Geraden-Kandidaten, die einen geschlossenen Pfad bilden und damit eine Blendenkontur-Kandidaten darstellen. Die Zahl dieser Blendenkontur-Kandidaten läßt sich gemäß Anspruch 7 deutlich einschränken. Dabei wird davon ausgegangen, daß der von einer Blendenkontur umschlossene Bereich stets höhere Bildwerte aufweist, als der von den Blenden abgedeckte Bereich.

Eine bevorzugte Möglichkeit zur Bestimmung der Konturpunkt-Kandidaten ist in Anspruch 8 angegeben. Statt dessen können die Konturpunkt-Kandidaten aber auch aus einem Hochpaßbild abgeleitet werden, in dem ebenfalls die Punkte hervorgehoben sind, die sich deutlich von ihren Nachbarn unterscheiden.

Eine bevorzugte Möglichkeit zur Ermittlung der Geraden-Kandidaten aus den Konturpunkt-Kandidaten ist in Anspruch 9 angegeben. Zwar können die Geraden-Kandidaten auch auf andere Weise ermittelt werden, z.B. mittels der linearen Regression, wie in der US-PS 5 651 042 beschrieben. Die Hough-Transformation bietet jedoch den Vorteil, daß eine Blendenkontur auch dann noch als Geraden-Kandidat erkannt wenn sie durch lange Bereiche unterbrochen wird, in denen sie praktisch keinen Kontrast im Röntgenbild zeigt.

Die in Anspruch 10 beschriebene Ausgestaltung bewirkt, daß das erfindungsgemäße Verfahren zur Ermittlung der Blendenkontur auch dann noch zu einem korrekten Ergebnis führt, wenn bei der Erstellung eines Röntgenbildes keine Blenden verwendet werden bzw. diese soweit geöffnet sind, daß ihre Konturen nicht im Röntgenbild dargestellt werden.

Es wurde gefunden, daß die Kontur eines Implantats sich von den Konturen anatomischer Strukturen im Röntgenbild einerseits durch einen größeren Kontrast andererseits aber auch durch einen geradlinigeren (d.h. einen glatteren oder einen weniger „rauhen") Verlauf der Kontur unterscheidet. Dies wird bei dem Verfahren nach Anspruch 11 ausgenutzt. Beide Auswahlkriterien (Kontrast und Geradlinigkeit) können in Kombination angewandt werden, jedoch ist es auch möglich, die Implantatkontur nur nach einem der beiden Kriterien auszuwählen. — Eine bevorzugte Möglichkeit zur Bestimmung des Geradlinigkeitsmaßes ist in Anspruch 12 angegeben.

Anspruch 13 beschreibt eine bevorzugte Anwendungsmöglichkeit des Verfahrens zur automatischen Detektion einer Implantatkontur.

Ein Röntgengerät zur Durchführung des erfindungsgemäßen Verfahrens ist in Anspruch 14 angegeben.

Die Erfindung wird nachstehend anhand der Zeichnungen näher erläutert.

Es zeigen:

Fig. 1          Ein Röntgengerät, mit dem die Erfindung ausführbar ist, in schematischer Darstellung,

Fig. 2          ein Ablaufdiagramm des erfindungsgemäßen Verfahrens,

Fig. 3a         das zu verarbeitende Eingangsbild,

Fig. 3b         die aus dem Eingangsbild abgeleiteten Geraden-Kandidaten,

Fig. 4a         einen Graphen, dessen Knoten durch die Schnittpunkte wenigstens zweier Geraden-Kandidaten gebildet werden und

Fig. 4b         die daraus abgeleitete Blendenkontur.

Fig. 5          stellt eine Nachbarschaftsmatrix dar, die die Berechnung der Linienintegrale entlang verschiedener geschlossener Pfade wesentlich erleichtert.

Fig. 6          eine Hough-Matrix

Fig. 7          eine Variante des in Fig. 2 dargestellten Verfahrens

Fig.8a und 8b   den dieser Variante zugrundeliegenden Bildwerteverlauf

Fig. 9a         ein Ablaufdiagramm zur automatischen Detektion einer Implantatkontur im Röntgenbild und

Fig. 10         einen Bildausschnitt mit geradlinigen und gekrümmten Abschnitten.

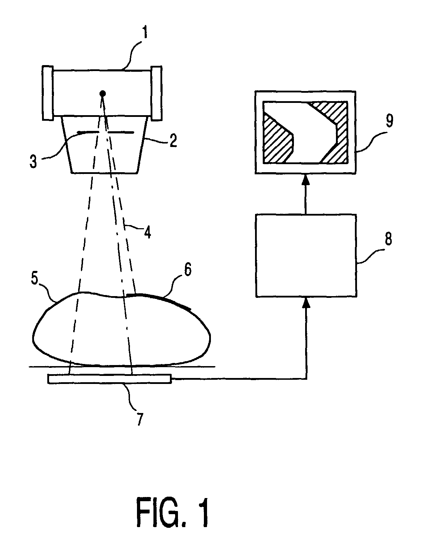

Das in Fig. 1 rein schematisch dargestellte Röntgenuntersuchungsgerät besitzt einen Röntgenstrahler 1, an dem eine Tiefenblende 2 befestigt ist. In der Tiefenblende befindet sich ein erstes Blendenplattenpaar 3 mit senkrecht zur Zeichenebene verlaufenden Blendenkanten zur Ausblendung eines Röntgenstrahlenbündels 4. Außerdem ist in der Tiefenblende 2 ein weiteres, nicht näher dargestelltes Blendenplattenpaar vorgesehen, dessen Kanten ebenfalls horizontal, aber parallel zur Zeichenebene der Fig. 1 verlaufen. Das Strahlenbündel 4 durchsetzt einen Untersuchungsbereich, in dem sich ein Patient 5 befindet. Ein Teil des Patienten kann durch eine geeignete Abdeckung 6 aus einem die Röntgenstrahlung absorbierenden Material z. B. einer Bleigummischürze vor dem Röntgenstrahlenbündel 4 abgeschirmt werden.

Hinter dem Patienten (vom Röntgenstrahler 1 aus gesehen) befindet sich ein digitaler Röntgenbildwandler, der das Röntgenbild in digitalisierte elektrische Signale umsetzt, die das Röntgenbild darstellen. Jedem Pixel des Röntgenbildwandlers ist dabei ein Bildwert zugeordnet, der um so größer ist, je geringer die Schwächung der Röntgenstrahlung durch das Untersuchungsobjekt 5 ist. Die von dem Röntgenbildwandler 7 erzeugten elektrischen Signale werden von einer Bildverarbeitungseinheit 8 erfaßt und weiterverarbeitet. Das dabei erzeugte Bild wird auf einem Monitor 9 dargestellt oder von einer nicht näher dargestellten Hardcopy-Einheit ausgegeben.

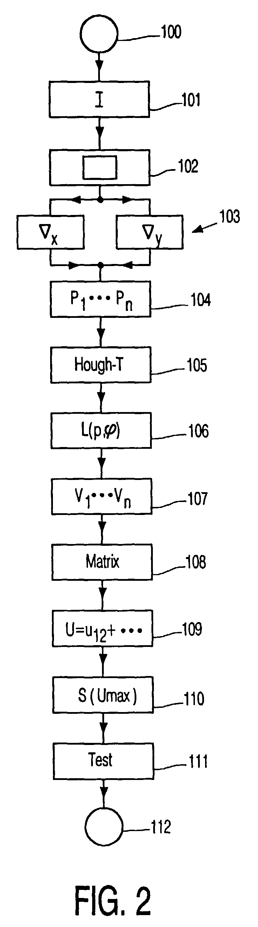

Das Ablaufdiagramm gemäß Fig. 2 erläutert die Verarbeitungsschritte, die in der Bildverarbeitungseinheit 8 ausgeführt werden, um die Blendenkontur zu detektieren.

Nach der Initialisierung (Block 100) erfolgt die Aufbereitung des Eingangsbildes im Schritt 101. Die nachfolgend beschriebene Aufbereitung ist an sich nicht notwendig, jedoch hat es sich als zweckmäßig erwiesen, das vom Bildwandler 7 gelieferte Eingangsbild wie folgt aufzubereiten:

a) Die für die einzelnen Pixel gelieferten Daten werden logarithmiert, so daß die Bildwerte nicht der Intensität der Röntgenstrahlung hinter dem Patienten 5 proportional sind, sondern dem Logarithmus dieser Intensität. Dadurch ergibt sich der gleiche Bildcharakter wie bei einer konventionellen Röntgenaufnahme auf einem Film.

b) Es erfolgt eine Unterabtastung des Bildes, d.h. statt eines Bildes mit z.B. 2000 x 2000 Pixeln wird ein Bild mit z.B. 500 x 500 Pixeln verarbeitet. Bei einer geringeren Unterabtastung des Eingangsbildes wächst der Rechenaufwand, und bei einer stärkeren Unterabtastung besteht die Gefahr, daß die Blendenkontur nicht mehr korrekt bestimmt werden kann. Diese Unterabtastung geht mit einer Glättung der Bildwerte einher.

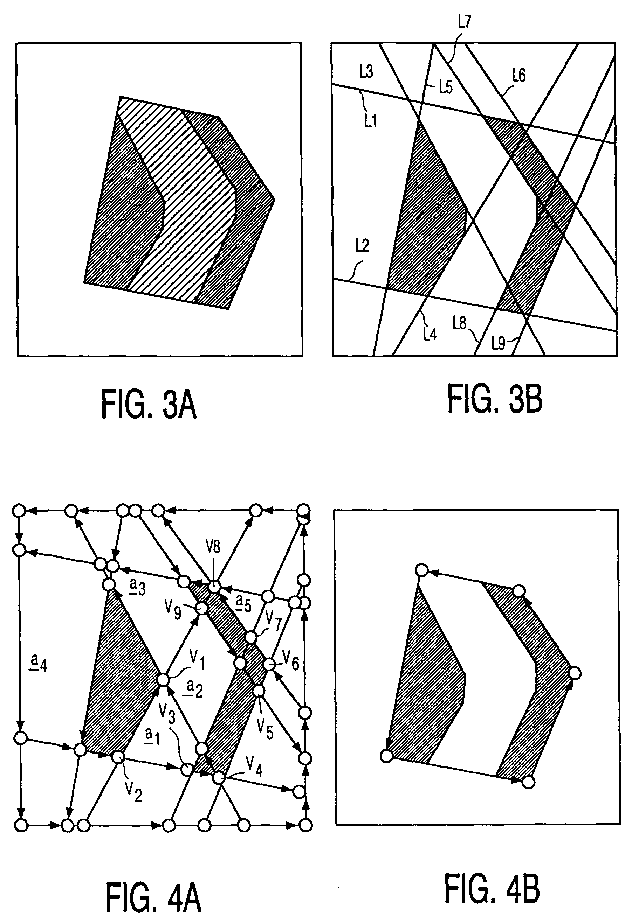

Im Schritt 102 wird das Eingangsbild, das in Fig. 3a dargestellt an seinen Außenrändern mit einem Rahmen versehen, d.h. an den Außenrändern werden Spalten bzw. Zeilen von Pixeln hinzugefügt, deren Bildwerte genauso groß sind wie die Bildwerte hinter einer Blende. Dadurch wird erreicht, daß die Bildbegrenzung auch dann korrekt ermittelt wird, wenn die Blenden 3 (Fig. 1) so weit aufgeblendet sind, daß sie nicht mehr im Röntgenbild abgebildet werden.

Im Schritt 103 werden aus dem (aufbereiteten) Eingangsbild zwei Bilder abgeleitet, die der x- bzw. y-Komponente des Gradienten des Eingangsbildes entsprechen. Bekanntlich ist der Gradient eines zweidimensionalen skalaren Feldes I (das Eingangsbild ist ein solches skalares Feld) ein Vektor

$$\nabla I = (\nabla_x I, \nabla_y I) \tag{1}$$

Für die Vektorkomponenten $\nabla_x I$ und $\nabla_y I$ gilt bekanntlich die Beziehung

$$\nabla_x I = {\partial I}/{\partial x} \, , \, \nabla_y I = {\partial I}/{\partial y} \tag{2}$$

d.h. die Vektorkomponenten stellen die räumliche Ableitung der Größe I (Bildwerte) in x- bzw. y-Richtung dar. Der Gradient wird auf diese Weise für sämtliche Pixel des Bildes ermittelt. Zur Berechnung der x-Komponente des Gradienten wird dabei das Bild mit einem eindimensionalen Kernel in x-Richtung gefaltet. Wenn mi Schritt 101 das Eingangsbild in geeigneter Weise gefiltert wurde, kann der Kernel aus den Komponenten -1,0,+1 bestehen, was darauf hinausläuft, daß jedem Pixel die Differenz zwischen dem Bildwert des rechten Nachbarpixels und des linken Nachbarpixels zugeordnet wird. Der Kernel kann auch größer sein. Wenn er jedoch zu groß ist, können sich wiederum Ungenauigkeiten ergeben und außerdem steigt der Rechenaufwand. Der y-Gradient kann in analoger Weise berechnet werden.

Es hat sich als zweckmäßig erwiesen, wenn die ermittelten Gradienten-Komponenten für die einzelnen Pixel mit dem (ggf. räumlich noch zusätzlich gemittelten) Bildwert I dieses Pixels gewichtet wird. Diese Gewichtung hat den gleichen Effekt, als würde man zunächst die Bildwerte quadrieren und dann den Gradienten nach der Beziehung

$$\nabla I^2 = 2 \, (I\nabla I_x, I\nabla I_y) \tag{3}$$

bilden. Die Gewichtung hat den Vorteil, daß Artefakte des Bildwandlers unterdrückt werden, weil sie hauptsächlich in den abgeblendeten Bildbereichen (niedrige Röntgenstrahlenintensität) sichtbar werden. Gleichzeitig werden dadurch die Blendenkanten, die Direkt-Strahlungsbereichen im Bild zugeordnet sind, durch die sich dort ergebenden hohen Bildwerte angehoben. Statt von $I^2$ kann man verallgemeinernd von $I^b$ ausgehen, wobei b eine Konstante >0. Für b=1 ergibt sich ein reines Gradientenbild.

Im Schritt 104 wird aus den für die einzelnen Pixel ermittelten (ggf. gewichteten) Gradienten die Konturpunkt-Kandidaten ermittelt. Alle Pixel, deren Gradient einen Betrag oberhalb eines bestimmten Schwellenwertes hat, werden als Konturpunkt-Kandidat betrachtet. Der Betrag des Gradienten ist bekanntlich die Quadratwurzel aus der Summe der Quadrate der einzelnen Komponenten des Gradienten. Anstelle dieser relativ aufwendigen Rechenoperation genügt es aber, wenn lediglich die Summe der Beträge herangezogen wird. Dieser Unterschied hat keine feststellbaren Auswirkungen auf die Genauigkeit der gefundenen Blendenkontur.

Im nächsten Schritt 105 wird zwecks Ermittlung der Geraden-Kandidaten eine Hough-Transformation durchgeführt und zwar eine Schnelle Hough-Transformation. Dabei wird jedem Konturpunkt mit den Koordinaten x,y eine Gerade zugeordnet, auf der dieser Punkt liegt und die den Steigungswinkel φ hat. Der Winkel φ berechnet sich nach der Formel

$$\varphi = \arctan \left( {\nabla_y}/{\nabla_x} \right) \tag{4}$$

$\nabla_x$ und $\nabla_y$ repräsentieren die Komponenten des Gradienten in x- bzw. y-Richtung in dem betreffenden Pixel. φ wird in einem Winkelbereich von -180° bis +180° ermittelt und zwar so, daß dieser Winkel zwischen -180° und -90° liegt, wenn die Komponenten $\nabla_x$ und $\nabla_y$ beide negativ sind, und zwischen 90° und 180°, wenn $\nabla_x$ positiv und $\nabla_y$ negativ ist. Der Abstand ρ der Geraden durch den Punkt x,y vom Koordinatenursprung errechnet sich dann nach der Beziehung

$$\rho = x \cos \varphi + y \sin \varphi \tag{5}$$

Dabei können sich auch negative Werte für $\rho$ ergeben. Geraden mit entgegengesetzt gleichen Werten von $\rho$ sind kollinear und antiparallel.

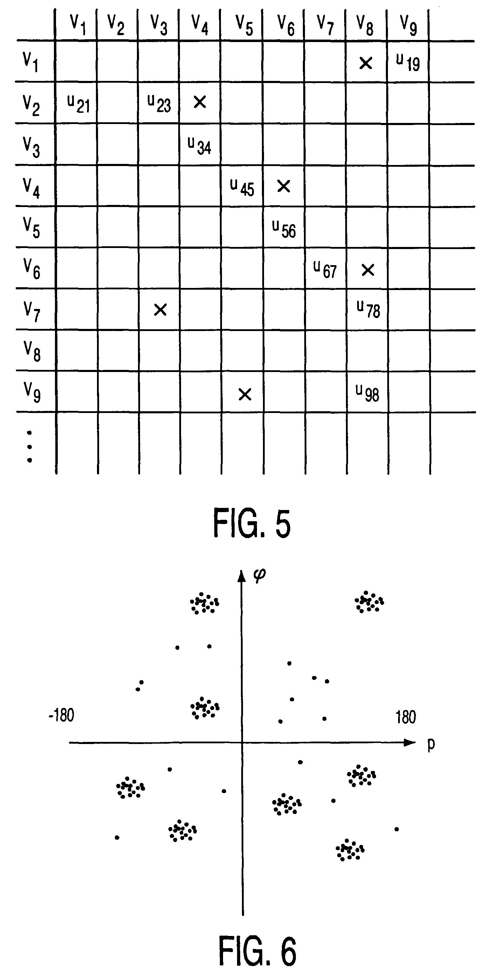

Durch die schnelle Hough-Transformation wird für jeden in der x,y-Ebene liegenden Punkt der Inhalt einer durch die Werte $\rho$ und $\varphi$ definerten Zelle in einer $\rho,\varphi$-Matrix um 1 erhöht. Eine typische $\rho,\varphi$-Matrix ist in Fig. 6 dargestellt. Wenn die Konturpunkt-Kandidaten auf einer Geraden liegen, müßte für jeden Punkt auf dieser Geraden der Inhalt der zugehörigen Zelle der $\rho$-$\varphi$-Matrix um den Wert 1 erhöht werden. Da in der Praxis der Wert $\rho,\varphi$ nicht exakt ermittelt werden kann, verteilen sich die Einträge auf die zu dem korrekten Wert $\rho,\varphi$ benachbarten Zeilen. Jedes Cluster von Zellen mit einem erhöhten Eintrag mi $\rho,\varphi$-Diagramm entspricht daher einem Geraden-Kandidaten. Demgemäß werden im Schritt 106 lokale Maxima in der $\rho,\varphi$-Matrix ermittelt, die jeweils einem Geraden-Kandidaten entsprechen.

In Fig. 3b sind die für das Eingangsbild nach Fig. 3a ermittelten Geraden-Kandidaten L1...L9 in einer x,y-Ebene dargestellt. Dazu können auch die Geraden gehören, die den äußeren Rand umrahmen. Wie zuvor erläutert, ist jeder Geraden ein Winkel im Bereich von -180° und bis +180° zugeordnet. Zwei Geraden, denen Winkel $\varphi$ zugeordnet sind, die sich um 180° unterscheiden, verlaufen antiparallel zueinander. Jedem Geraden-Kandidaten ist somit eine Richtung zugeordnet. Rechts (in Richtung der Geraden gesehen) von der Geraden befinden sich die Pixel mit einer geringeren Röntgenstrahlenintensität und links davon die Pixel, die bei der Röntgenaufnahme einer höheren Strahlenintensität ausgesetzt waren (dies gilt für die angegebenen eindimensionalen Kernel für die Berechnung der Gradienten-Komponenten $\nabla_x$ und $\nabla_y$, bei denen die Bildwerte des linken bzw. oberen Pixels mit einem negativen Gewicht eingehen.).

Im nächsten Schritt 107 werden zunächst die Schnittpunkte $V_1 \dots V_n$ aller Geraden-Kandidaten berechnet. Zumindest einige dieser Schnittpunkte sind Eckpunkte des durch die Blendenkontur definierten Polygons. In Fig. 4a sind einige dieser Schnittpunkte bzw. möglichen Eckpunkte mit $V_1...V_9$ bezeichnet. Außerdem ist die zuvor erläuterte Richtung der Geraden durch Pfeile angedeutet. Eine Sequenz solcher Eckpunkte wird als Pfad bezeichnet. Wenn bei einem Pfad der erste und der letzte Eckpunkt zusammenfallen, wird der Pfad als geschlossen (cyclic) bezeichnet.

Die Erfindung geht davon aus, daß die Blendenkontur ein Polygon (also ebenfalls ein geschlossener Pfad) ist, das aus Geraden-Kandidaten oder einzelnen Stücken davon zusammengesetzt ist. Die Erfindung basiert auf der Annahme, daß die Kontraste entlang der Blendenkontur maximal sind. Es besteht somit die Aufgabe, aus der Vielzahl der möglichen geschlossenen Pfade denjenigen geschlossenen Pfad zu finden, entlang dessen sich der größtmögliche Kontrast ergibt.

Eine erste Möglichkeit zur Bestimmung des Konrastverlaufs entlang eines geschlossenen Pfades besteht in der Berechnung des Umlaufintegrales $U_{tot}$ nach der Beziehung

$$U_{tot} = \oint (n \, \nabla I^b) \, ds \tag{6}$$

Dabei stellt n einen Vektor der Länge 1 dar, der jeweils senkrecht zu dem geschlossenen Pfad verläuft. $U_{tot}$ stellt somit das Umlaufintegral des (mit I gewichteten), auf die Normale zu dem geschlossenen Pfad projizierten Gradienten dar. Das Umlaufintegral $U_{tot}$ kann als Summe von Teilintegralen dargestellt werden, die gemäß der Beziehung

$$U_{tot} = \Sigma \, u_i \tag{7}$$

berechnet werden. Dabei ist $u_i$ gemäß der Gleichung

$$u_i = \int_{si} (n \, \nabla I^b) \, ds \tag{8}$$

die Richtungsableitung des Gradienten $\nabla I^b$ entlang eines durch zwei aufeinanderfolgende, auf dem gleichen Geraden-Kandidaten liegende Schnittpunkte (z.B. $V_1$, $V_2$) definierten Pfades.

Zur Verringerung der Rechenzeit werden dabei nur solche geschlossenen Pfade untersucht, bei denen alle Stücke des geschlossenen Pfades im Gegenuhrzeigersinn irgendeinen Bereich des Eingangsbildes umschließen. Dieser Bereich beinhaltet Pixel mit einer höheren Röntgenstrahlenintensität als die Pixel außerhalb dieses Bereiches. Von den auf diese Weise immer noch möglichen geschlossenen Pfaden wird derjenige als Blendenkontur ausgewählt, entlang dessen die größten Kontraste akkumuliert werden können.

Um zu vermeiden, daß der Wert $u_i$ mehrfach für einen Pfad $s_i$ berechnet wird, der Bestandteil mehrer geschlossener Pfade ist, werden im Schritte 108 die Werte $u_i$ gemäß Gleichung 8 einmal berechnet und in eine Nachbarschaftsmatrix (englisch: adjacency matrix) übernommen werden. Fig. 5 stellt eine solche Nachbarschaftsmatrix dar, in die sämtliche Eckpunkte V1, V2...V9........... eingetragen werden müssen. Dabei erfolgt nur für auf der selben Geraden liegende Eckpunkte ein Eintrag. In der Zeile für V2 gibt es dabei einen Eintrag $u_{21}$, der sich auf den von V2 nach V1 gerichteten Pfad bezieht. Ebenso gibt es einen Eintrag $u_{23}$ für den von V2 nach V3 gerichteten Pfad.

Es wäre auch möglich, für den Pfad von V2 nach V4 einen Eintrag vorzusehen (was in der Zeile von V2 und der Spalte von V4 durch ein Kreuz angedeutet ist), doch ist dies nicht notwendig. Hingegen gibt es keinen Eintrag für den Pfad von V2 nach V5, weil V2 und V5 nicht auf einer Geraden liegen. Außerdem gibt es keinen Eintrag von V2 zu den Punkten links bzw. unterhalb von V2, die auf der selben Gerade liegen wie V2, weil der Pfad von V2 aus nicht in diese Richtung zeigt (für die von diesen nicht näher bezeichneten Eckpunkten zu V2 führenden Pfade gibt es jedoch einen Eintrag in der Zeile für die betreffenden Punkte und in der Spalte für V2).

Im Schritt 109 wird daher das Umlaufintegral gemäß Gleichung 7 aus den Einträgen für die Pfade berechnet, aus denen sich der geschlossene Pfad zusammensetzt. Dies wird für sämtliche möglichen Pfade wiederholt, die einen Bereich im Gegenuhrzeigersinn umschließen.

Im Block 110 wird dann derjenige geschlossene Pfad als Blendenkontur ausgewählt, für den $U_{tot}$ größer ist als für sämtliche anderen geschlossenen Pfade. Die so gefundene Blendenkontur ist in Fig. 4b dargestellt.

Im Schritt 111 kann die auf diese Weise ermittelte Blendenkontur einem Plausibilitätstest unterzogen werden (wegen der hohen Zuverlässigkeit dieses verfahrens kann dieser Schritt aber auch entfallen). Dabei wird der Medianwert der Bildwerte der Pixel in dem von der Blendenkontur umschlossenen Bereich gebildet. Dann wird die Nummer der Pixel gezählt, die außerhalb des von dem Pfad umschlossenen Bereiches liegen und deren Bildwert größer ist als der so berechnete Medianwert. Die Zahl dieser anscheinend zu hellen Pixel wird durch die Zahl der Pixel in dem von der Blendenkontur umschlossenen Bereich dividiert. Wenn die so ermittelte Anzahl der "Ausreißer" einen gewissen Schwellwert, z.B. 30% überschreitet, wird die in den Schritten 101 bis 110 gefundene Blendenkontur wieder verworfen. Theoretisch müßten alle durch die Blende abgedeckten Pixel einen Bildwert haben, der niedriger ist als der Medianwert in dem von der Blende freigelassenen Bereich. Infolge von Rauschen oder Artefakten des Bildwandlers kann es jedoch eine Anzahl von Ausreißern geben, die einen höheren Bildwert haben als der Medianwert. Wenn dieser Anteil aber zu groß wird, ist dies ein Indiz dafür, daß die gefundene Blendenkontur nicht richtig ermittelt worden ist. In diesem Fall wird die gefundene Blendenkontur durch den Rahmen ersetzt, der im Schritt 102 hinzugefügt ist.

Damit ist die Detektion der Blendenkontur beendet (Block 112).

Eine zweite Möglichkeit zur Bestimmung des entlang eines geschlossenen Pfades akkumulierten Kontrasts besteht in der Berechnung des Flächenintegrals $V_{tot}$ des Laplace-Operators in dem von dem geschlossenen Pfad begrenzten Bereich nach der Beziehung

$$V_{tot} = \int \Delta I^b \, da. \tag{9}$$

$\Delta$ stellt dabei den Laplace-Operator dar, für den sich in der angelsächsischen Literatur die Bezeichnung $\nabla^2$ eingebürgert hat. Der Laplace-Operator $\Delta$ einer von x und y abhängigen Funktion f berechnet sich bekanntlich nach der Beziehung

$$\Delta f = \partial^2 f / \partial x^2 + \partial^2 f / \partial y^2 \tag{10}$$

Obwohl die Formel für $V_{tot}$ nach Gleichung 9 auf den ersten Blick stark von der Formel für $U_{tot}$ nach Gleichung 6 abweicht, läßt sich mit Hilfe des Divergenztheorems zeigen, daß beide Gleichungen mathematisch identisch sind.

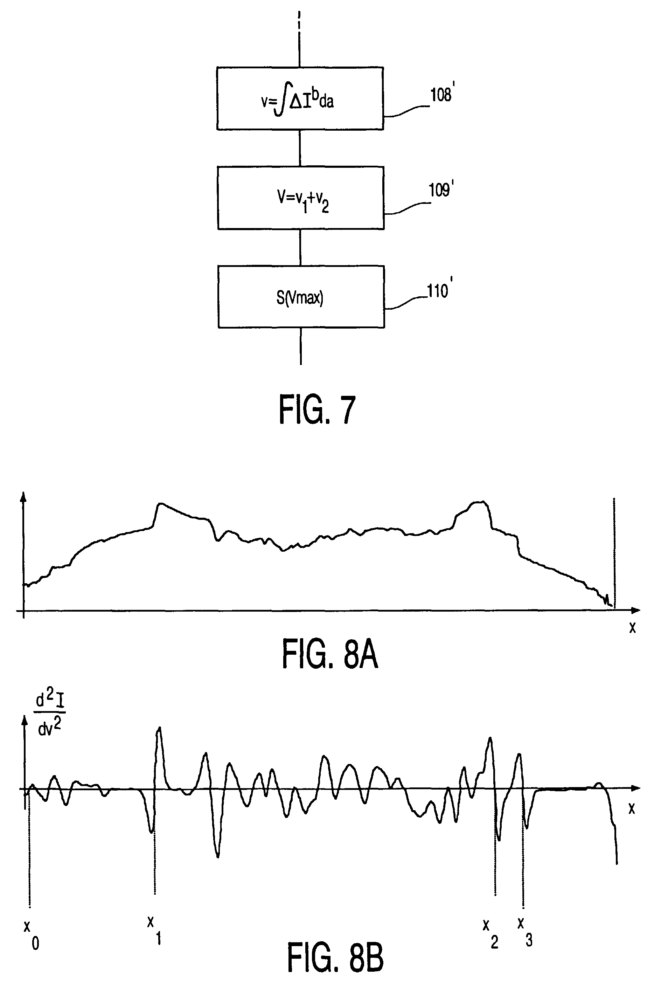

Eine vereinfachte Deutung dieses Ansatzes nach Gleichung 9 ist in Fig. 8 dargestellt. Dabei zeigt Fig. 8a einen typischen Verlauf von Bildwerten in x-Richtung und Fig. 8b die zugehörige zweite Ableitung (die für diesen eindimensionalen Fall den Laplace-Operator darstellt). Blendenkanten erscheinen dabei in dem Bild nach Fig. 8b durch besonders große Werte der zweiten Ableitung, wobei ein positiver Wert durch einen negativen Wert in etwa der gleichen Größe kompensiert wird. Bildet man nun das Integral über die zweite Ableitung in x- Richtung zwischen den Nulldurchgängen $x_1$ und $x_2$, dann wird dieses Integral (in das das Integral gemäß Gleichung 9 für den eindimensionalen Fall übergeht) im wesentlichen durch die positiven Amplituden bei $x_1$ und $x_2$ bestimmt. Die dazwischen liegenden Kurventeile mitteln sich im wesentlichen heraus. Die zweite Ableitung hat noch weitere (relative) Maxima bzw. Minima z.B. bei $x_0$ und $x_3$. Da diese jedoch kleiner sind als die bei $x_1$ und $x_2$, würde das Integral über die zweite Ableitung von $x_0$ bis $x_3$ einen kleineren Wert ergeben als von $x_1$ bis $x_2$.

Auf diesen Überlegungen aufbauend, zeigt nun Fig. 7 einen modifizierten Teil des Ablaufdiagramms nach Fig. 2, der an die Stelle der dort vorgesehenen Schritte 108 bis 110 tritt.

Gemäß Fig. 4a lassen sich sämtliche bei der gegebenen Lage der Eckpunkte $V_1...V_9$...möglichen Polygone aus einem oder mehreren Elementarpolygonen zusammensetzen, von denen einige in Fig. 4a mit $a_1...a_2...a_5$ bezeichnet sind. Für jedes dieser Elementarpolygone wird im Schritt 108' das Flächenintegral über den Laplace Operator der Bildwerte gemäß Gleichung 9 berechnet, so daß sich z.B. für $a_1$ ein Wert $v_1$, für $a_2$ ein Wert $v_2$ usw. ergibt. Der Laplace-Operator für jedes einzelne Pixel läßt sich aus den im Schritt 103 berechneten Gradienten ableiten, indem wiederum

EP 1 037 166 A1

einerseits die Komponenten $\nabla_x I$ in diesem und in den in x-Richtung benachbarten Pixeln und andererseits die Komponenten $\nabla_y I$ in diesem und in den in y-Richtung benachbarten Pixeln mit einem Kernel -1,0,+1 gefaltet werden und die Faltungsprodukte addiert werden.

Jeder geschlossene Pfad umschließt ein Polygon, das sich aus einem oder mehreren dieser Elementarpolygone zusammensetzt. Im Schritt 109' werden daher für die Elementarpolygone ($a_1$, $a_2$, $a_5$ usw.), die von dem geschlossenen Pfad umschlossen worden, die zugehörigen Werte $v_1$, $v_{2...}v_5$ usw. addiert, so daß sich der zu dem geschlossenen Pfad gehörender Gesamtwert $V_{tot}$ ergibt.

Im Schritt 110' wird dann von den auf diese Weise bewerteten geschlossenen Pfaden derjenige als Blendenkontur selektiert, für den der Wert $V_{tot}$ ein Maximum erreicht. Die so gefundene Blendenkontur kann gemäß Fig. 2, Schritt 111 wiederum einem Plausibilitätstests unterzogen werden, bevor das Verfahren gemäß Block 112 endet.

Auch bei diesem Verfahren sind wiederum Möglichkeiten zur Reduktion der Rechenzeit gegeben. Je größer nämlich die Zahl der gefundenen Geraden-Kandidaten ist, desto größer ist auch die Zahl der geschlossenen Pfade, unter denen derjenige ermittelt werden muß, der die Blendenkontur repräsentiert. Bei N Geraden-Kandidaten gibt es $2^N$ geschlossene Pfade. Bei sehr großen Werten von N ist es kaum möglich, den Wert $V_{tot}$ für jeden einzelnen Pfad zu berechnen.

Deshalb könnte der gesuchte Pfad mit wesentlich geringerem Rechenaufwand wie folgt ermittelt werden: Ausgehend von einem Vektor, der so viele Komponenten aufweist, wie Geraden-Kandidaten vorhanden sind (N), wird jedem Geraden-Kandidaten eine Vektor-Komponente zugeordnet mit einem Wert, der etwas darüber aussagt, ob dieser Geraden-Kandidat Teil der Blendenkontur ist (in diesem Fall hat die Komponente den Wert 1) oder nicht (in diesem Fall ist der Wert für die Komponente 0). Ausgehend von einem Startvektor, bei dem beispielsweise alle Komponenten auf 0 gesetzt werden, wird untersucht, für welchen einzelnen Gerade-Kandidaten sich der maximale Wen $V_{tot}$ ergibt (dazu müssen die Werte $v_1$, $v_{2...}$ usw. von allen Elementarpolygonen $a_1$, bzw. $a_2$ usw. addiert werden, die — in Richtung des zu dem Geraden-Kandidaten gehörenden Pfeils gesehen — links von diesem Geraden-Kandidaten liegen). Der Geraden-Kandidat, für den sich der größte Wert $V_{tot}$ ergibt wird invertiert (von 0 auf 1 gesetzt oder von 1 auf 0). Danach wird das Verfahren wiederholt, indem die Vektor-Komponenten für alle Geraden-Kandidaten invertiert werden und geprüft wird, welche einzelne Invertierung dem größten Wert $V_{tot}$ führt. Dies wird so oft wiederholt, bis das Flächenintegral über den Laplace-Operator ($V_{tot}$) nicht weiter zunimmt.

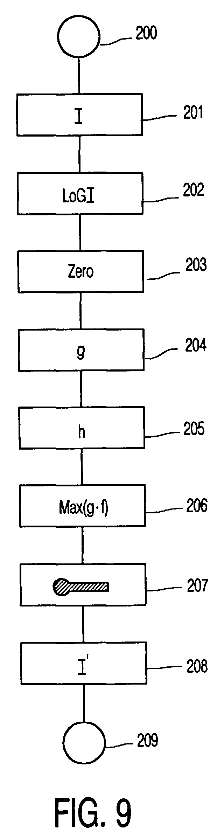

Anhand des Ablaufdiagramms in Fig. 9 wird im folgenden ein Verfahren zur automatischen Detektion der Kontur eines Implantats erläutert.

Nach der Initialisierung im Block 200 erfolgt zunächst im Schritt 201 eine Aufbereitung des Röntgenbildes — wie anhand des Verfahrensschrittes 101 bei dem Ablaufdiagramm nach Fig. 2 erläutert. Daraus resultiert ein Bild mit Bildwerten I, die eine Funktion des Ortes x,y sind.

Im Schritt 202 wird auf das so aufbereitete Bild I ein sogenanntes LoG-Filter (LoG=Laplacian of Gaussian) angewendet. Das LoG-Filter ist ein wohl bekanntes Bildverarbeitungsfilter, welches die zweite räumliche Ableitung der Bildwerte nach einer Glättung mit einem Gauß-Tiefpaßfilter gemäß der Beziehung

$$I_{LoG} = \Delta I^b(x,y) \exp\left(-\frac{(x^2+y^2)}{2\sigma^2}\right) \qquad (11)$$

darstellt, wobei $I_{LoG}$ die so gefilterten Bildwerte darstellen und $\sigma$ ein Parameter, der die Weite der Gauß-Tiefpaßfilterung darstellt. In guter Näherung kann man eine solche Filterung durch Subtraktion zweier Gauß-Filterungen mit unterschiedlicher Kernelgröße realisieren (DoG-Filter; DoG=Difference of Gaussians). Eine Kontur im Röntgenbild entspricht einem Null-Durchgang in dem LoG-gefilterten Bild. (Als Null-Durchgang wird der Wechsel des Vorzeichens gezeichnet). Die Konturlinien, die sich ergeben, wenn man den Null-Durchgängen in dem LoG-gefilterten Bild folgt, haben die bekannte Eigenschaft, daß sie immer geschlossen sind. Von irgendeinem Pixel ausgehend, in dem sich so ein Null-Durchgang vollzieht, gelangt man, wenn man den sich anschließenden Null-Durchgängen folgt wieder zu diesem Pixel zurück. Eine weitere bekannte Eigenschaft des LoG-gefilterten Bildes ist, daß sich die auf diese Weise ergebenden geschlossenen Pfade niemals schneiden. Somit können auf diese Weise im Schrift 203 die Konturen im Eingangsbild bzw. in dem Röntgenbild detektiert werden.

Dieses Verfahren ist einfacher als wenn man zunächst Konturpunkte bestimmt, danach Geraden, die diese Konturpunkte verbinden und aus den Geraden schließlich geschlossene Pfade zusammensetzt, von denen einer die gesuchte Kontur darstellt. Gleichwohl ist es nicht ratsam, dieses Verfahren auch auf die automatische Detektion von Blendenkonturen anzuwenden, weil die Blendenkontur in einem Röntgenbild sehr oft Abschnitte aufweist, die wegen ihres geringen Kontrastes kaum zu identifizieren sind und weil in diesem Fall eine Verfolgung der Null-Durchgänge nicht mehr möglich ist.

Unter den auf diese Weise gefundenen Konturen muß wiederum die ausgewählt werden, die mit der größten Wahrscheinlichkeit die Kontur eines Implantats wiedergibt. Bei der Ermittlung dieser Kontur wird davon ausgegangen, daß sie einen stärkeren Mittelwert des Kontrastes aufweist als die anderen Konturen und daß sie außerdem ein höheres Maß an Geradlinigkeit aufweist als Konturen von anatomischen Objekten. Dabei wird davon ausgegangen, daß ein künstliches Implantat im Röntgenbild eine geradere, zumindest aber eine glattere Kontur aufweist als eine natürliche Struktur.

Dementsprechend wird im Schritt 204 der mittlere Kontrast und im Schritt 205 ein Maß für die Geradlinigkeit der Kontur ermittelt. Der Kontrast g kann nach der folgenden Gleichung ermittelt werden:

$$g = \frac{i_{out} - i_{in}}{i_{out} + i_{in}} \tag{12}$$

Dabei ist $i_{in}$ der Mittelwert der Bildwerte an der Innenseite der Kontur und $i_{out}$ der Mittelwert der Bildwerte an der Außenseite der Kontur. Eine andere Möglichkeit besteht darin, die Werte $U_{tot}$ nach Gleichung 6 bzw. $V_{tot}$ nach Gleichung 9 zu berechnen und durch die Länge der Kontur zu dividieren.

Für jedes Pixel $P_i$ auf der Kontur berechnet sich ein Geradlinigkeitsmaß $h_i$ aus den Koordinaten von N benachbarten Pixeln auf der Kontur nach der Beziehung

$$h_i = d/N,$$

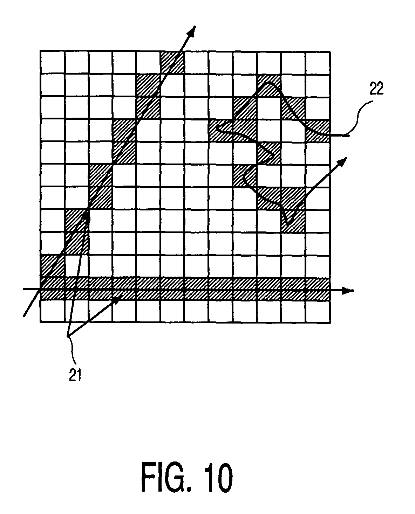

wobei d das Maximum aus den Abständen dx, dy des ersten dieser Pixel in x- und y-Richtung vom letzten der N-Pixel ist. Fig. 10 veranschaulicht die Bedeutung dieses Maßes. Darin sind zwei — zumindest über eine bestimmte Anzahl von Pixel hinweg — gerade Konturen 21 dargestellt. Der Abstand dx des ersten und des letzten Pixels auf der horizontalen Korrektur — gemessen in Pixelbreiten — entspricht der Zahl N der Pixel, so daß das Maß $h_i$ hier den Wert 1 annimmt. Bei der schräg verlaufenden geraden Linie ist der Abstand d (in diesem Fall ist d=dy ) zwar etwas geringer, so daß auch der Wert $h_i$ geringer ist, jedoch ist er auch dabei stets größer als 0,7. Bei der gekrümmten Kontur 22 hingegen beträgt d (in diesem Fall ist ebenfalls d=dy ) nur 2 (Pixelbreiten), so daß sich ein niedriger Wert für $h_i$ ergibt.

Das Geradlinigkeitsmaß h für die gesamte Kontur ergibt sich als Mittelwert aus den Maßen $h_i$ in den einzelnen Pixeln der Kontur. Durch die Zahl N der Pixel, die bei der Berechnung von $h_i$ herangezogen werden, kann das Geradlinigkeitsmaß h bzw. $h_i$ den Bedürfnissen angepaßt werden. Je kleiner die Zahl N ist, desto mehr nimmt bei einer gekrümmten Kurve der Wert $h_i$ zu. Deshalb wird auch eine glatte, gleichmäßig gekrümmte Kontur als geradlinig bewertet, wenn die durch N aufeinanderfolgende Pixel definierte Länge klein ist im Vergleich zum Krümmungsradius dieser Kontur.

Im Schritt 206 wird dann die Kontur ausgewählt, für die das Produkt aus den in den Schritten 204 und 205 ermittelten Werten von g und h maximal ist. Diese Kontur stellt die gesuchte Kontur des Implantats dar. Im Schritt 207 wird der von der gefundenen Kontur des Implantats umschlossene Bereich eliminiert, und im Schritt 208 werden aus dem verbleibenden Restbild in bekannter Weise die Parameter für die automatische Einstellung der Kontraste auf einem Monitor (bzw. in einer Hard-Copy-Einheit) ermittelt, mit deren Hilfe das Röntgenbild mit optimalem Kontrast wiedergegeben werden kann.

**Patentansprüche**

1. Verfahren zur automatischen Detektion von Konturen von Strukturen mit hoher Röntgenstrahlenabsorption in einem Röntgenbild
   gekennzeichnet durch die Schritte

   a) Ermittlung einer Anzahl von geschlossenen Pfaden in einem Röntgenbild oder einem daraus abgeleiteten Bild als Kontur-Kandidaten,
   b) Auswahl eines geschlossenen Pfades — aus der Anzahl von geschlossenen Pfaden — als Kontur in Abhängigkeit von den Kontrasten entlang der geschlossenen geschlossenen Pfade.

2. Verfahren nach Anspruch 1 zur Ermittlung einer Blendenkontur,
   dadurch gekennzeichnet, daß die folgenden Schritte zur Ermittlung der geschlossenen Pfade durchgeführt werden

   a) Bestimmung von Konturpunkt-Kandidaten aus dem räumlichen Verlauf der den Pixeln des Röntgenbildes oder eines daraus abgeleiteten Eingangsbildes zugeordneten Bildwerten,
   b) Bestimmung von Geraden-Kandidaten aus den Konturpunkt-Kandidaten derart, daß auf jedem Geraden-Kandidaten - oder unmittelbar daneben - sich eine Reihe von Konturpunkt-Kandidaten befindet,

c) Bildung von geschlossenen Pfaden als Blendenkontur-Kandidaten, die aus Stücken verschiedener Geraden-Kandidaten zusammengesetzt sind,

d) Auswahl eines geschlossenen Pfades als Blendenkontur in Abhängigkeit von den Kontrasten entlang der geschlossenen Pfade.

3. Verfahren nach Anspruch 1 zur Ermittlung der Kontur eines Implantats,
dadurch gekennzeichnet, daß die folgenden Schritte zur Ermittlung der geschlossenen Pfade durchgeführt werden:

a) Anwendung eines LoG-Filters auf ein Röntgenbild oder einem daraus abgeleitetes Bild,
b) Ermittlung der geschlossenen Pfade, auf denen sich die Nulldurchgänge des LoG gefilterten Bildes befinden.

4. Verfahren nach Anspruch 2,
dadurch gekennzeichnet, daß der Auswahlschritt die Summierung der Kontraste an den Rändern beiderseits eines von mehreren geschlossenen Pfades und die Bestimmung des geschlossenen Pfades als Blendenkontur umfaßt, entlang dessen die Summe maximal ist.

5. Verfahren nach Anspruch 4,
dadurch gekennzeichnet, daß zur Summierung der Kontraste das Kurvenintegral ($U_{tot}$) entlang des geschlossenen Pfades (s) über die Richtungsableitung des Gradienten ($\nabla I$) der Bildwerte (I) in Richtung des Pfades (s) nach der Beziehung

$$U_{tot} = \oint (n \, \nabla I^a) \, ds \, ,$$

gebildet wird, wobei $n^t$ einen zu dem geschlossenen Pfad (s) senkrechten Einheitsvektor darstellt und a ein Exponent $\geq 1$ ist.

6. Verfahren nach Anspruch 4
dadurch gekennzeichnet, daß zur Summierung der Kontraste das Integral ($V_{tot}$) über den Laplace-Operator ($\Delta$) in dem von dem geschlossenen Pfad umschlossenen Bereich (a) gebildet wird nach der Beziehung:

$$V_{tot} = \int \Delta I^b da$$

7. Verfahren nach Anspruch 2,
gekennzeichnet durch die Beschränkung der Auswahl auf solche geschlossenen Pfade, die je einen Bereich umschließen, innerhalb dessen die Bildwerte größer sind als außerhalb dieses Bereiches.

8. Verfahren nach Anspruch 2,
gekennzeichnet durch die Auswahl derjenigen Punkte im Röntgenbild bzw. im Eingangsbild als Konturpunkt-Kandidaten, in denen die Bildwerte die größten Gradienten aufweisen.

9. Verfahren nach Anspruch 2,
gekennzeichnet durch die Ableitung der Geraden-Kandidaten aus den Konturpunkt-Kandidaten mittels einer Hough-Transformation.

10. Verfahren nach Anspruch 2,
gekennzeichnet durch die Vorgabe eines Rahmens um das Eingangsbild, wobei die den Pixeln auf diesem Rahmen den Bildwerten in dem durch Blenden abgeschirmten Bereich entsprechen.

11. Verfahren nach Anspruch 1,
gekennzeichnet durch die automatische Auswahl eines geschlossenen Pfades — aus der Anzahl von geschlossenen Pfaden — als Implantat-Kontur in Abhängigkeit von einem die Geradlinigkeit der geschlossenen Pfade bewertenden Geradlinigkeitsmaß.

12. Verfahren nach Anspruch 11
dadurch gekennzeichnet, daß zur Bestimmung des Geradlinigkeitsmaßes der Abstand zwischen dem ersten und dem letzten Pixel auf einem eine Anzahl benachbarter Pixel umfassenden Abschnitt eines geschlossenen Pfades in Relation zu der Länge dieses Abschnitts gesetzt wird.

13. Verfahren nach Anspruch 1,
gekennzeichnet durch die Unterdrückung des von der Implantatkontur umschlossenen Bereiches bei der automatischen Kontrasteinstellung in Abhängigkeit vom Inhalt des Röntgenbildes.

14. Röntgengerät zur Durchführung des Verfahrens nach Anspruch 1 mit

- einem Röntgenstrahler zur Erzeugung von einen Untersuchungsbereich durchsetzenden Röntgenstrahlen,
- einer zwischen dein Röntgenstrahler und dem Untersuchungsbereich befindlichen Blendenanordnung
- einem Röntgen-Bildwandler zur Erfassung der Röntgenstrahlung hinter dem Untersuchungsbereich und zur Erzeugung eines entsprechenden Eingangsbildes
- und einer Bildverarbeitungseinheit zur Detektion der Konturen der Blendenanordnung im Eingangsbild,

dadurch gekennzeichnet, daß die Bildverarbeitungseinheit zur Ausführung der folgenden Verarbeitungsschritte ausgebildet ist:

a) Bestimmung von Konturpunkt-Kandidaten aus dem räumlichen Verlauf der den Pixeln des Röntgenbildes oder eines daraus abgeleiteten Eingangsbildes zugeordneten Bildwerten,
b) Bestimmung von Geraden-Kandidaten aus den Konturpunkt-Kandidaten derart, daß auf jedem Geraden-Kandidaten - oder unmittelbar daneben - sich eine Reihe von Konturpunkt-Kandidaten befindet
c) Bildung einer Anzahl von geschlossenen Pfaden als Blendenkontur-Kandidaten, die aus Stücken verschiedener Geraden-Kandidaten zusammengesetzt sind,
d) Auswahl eines geschlossenen Pfades — aus der Anzahl von geschlossenen Pfaden — als Blendenkontur in Abhängigkeit von den Kontrasten beiderseits der geschlossenen Pfade.

FIG. 1

100

101 I

102

103 $\nabla_x$ $\nabla_y$

104 $P_1 \cdots P_n$

105 Hough-T

106 $L(p,\varphi)$

107 $V_1 \cdots V_n$

108 Matrix

109 $U = u_{12} + \cdots$

110 $S(U_{max})$

111 Test

112

FIG. 2

FIG. 3A

FIG. 3B

FIG. 4A

FIG. 4B

|     | $V_1$ | $V_2$ | $V_3$ | $V_4$ | $V_5$ | $V_6$ | $V_7$ | $V_8$ | $V_9$ |     |
|-----|-------|-------|-------|-------|-------|-------|-------|-------|-------|-----|
| $V_1$ |     |       |       |       |       |       |       | ✕ | $u_{19}$ |   |
| $V_2$ | $u_{21}$ |   | $u_{23}$ | ✕ |       |       |       |       |       |   |
| $V_3$ |     |       |       | $u_{34}$ |   |       |       |       |       |   |
| $V_4$ |     |       |       |       | $u_{45}$ | ✕ |       |       |       |   |
| $V_5$ |     |       |       |       |       | $u_{56}$ |   |       |       |   |
| $V_6$ |     |       |       |       |       |       | $u_{67}$ | ✕ |       |   |
| $V_7$ |     |       | ✕ |       |       |       |       | $u_{78}$ |       |   |
| $V_8$ |     |       |       |       |       |       |       |       |       |   |
| $V_9$ |     |       |       |       | ✕ |       |       | $u_{98}$ |       |   |

## FIG. 5

## FIG. 6

$$v = \int \Delta I^b \, da$$ —108'

$$V = v_1 + v_2$$ —109'

$$S(Vmax)$$ —110'

## FIG. 7

## FIG. 8A

$\dfrac{d^2 I}{dv^2}$

$x_0$    $x_1$    $x_2$   $x_3$

## FIG. 8B

**FIG. 9**

FIG. 10

Europäisches
Patentamt

**EUROPÄISCHER RECHERCHENBERICHT**

Nummer der Anmeldung

EP 00 20 0798

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| A | US 4 843 630 A (CATROS JEAN YVES  ET AL) 27. Juni 1989 (1989-06-27) * Zusammenfassung * * Spalte 1, Zeile 40 – Zeile 54 * * Spalte 4, Zeile 6 – Zeile 64 * --- | 1-14 | G06T5/00 |
| A | EP 0 609 922 A (KONINKL PHILIPS ELECTRONICS NV) 10. August 1994 (1994-08-10) * Zusammenfassung * * Seite 5, Zeile 6 – Zeile 14 * --- | 1-14 | |
| A | US 5 072 384 A (NAKAMORI NOBUYUKI  ET AL) 10. Dezember 1991 (1991-12-10) * Zusammenfassung; Ansprüche 5-7,32-35; Abbildungen 8A-8C * ----- | 1-14 | |

|  |  |
|---|---|
|  | **RECHERCHIERTE SACHGEBIETE (Int.Cl.7)** |
|  | G06T |

Der vorliegende Recherchenbericht wurde für alle Patentansprüche erstellt

| Recherchenort | Abschlußdatum der Recherche | Prüfer |
|---|---|---|
| DEN HAAG | 27. Juni 2000 | Gonzalez Ordonez, O |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus anderen Gründen angeführtes Dokument
.........................................................................
& : Mitglied der gleichen Patentfamilie, übereinstimmendes
    Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**          EP 00 20 0798

In diesem Anhang sind die Mitglieder der Patentfamilien der im obengenannten europäischen Recherchenbericht angeführten
Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

27-06-2000

| Im Recherchenbericht angeführtes Patentdokument | | Datum der Veröffentlichung | Mitglied(er) der Patentfamilie | | Datum der Veröffentlichung |
|---|---|---|---|---|---|
| US 4843630 | A | 27-06-1989 | FR | 2590702 A | 29-05-1987 |
| | | | DE | 3685571 A | 09-07-1992 |
| | | | DE | 3685571 T | 21-01-1993 |
| | | | EP | 0229543 A | 22-07-1987 |
| EP 0609922 | A | 10-08-1994 | US | 5365429 A | 15-11-1994 |
| US 5072384 | A | 10-12-1991 | DE | 3938699 A | 31-05-1990 |
| | | | JP | 2220638 A | 03-09-1990 |
| | | | JP | 2796381 B | 10-09-1998 |

Für nähere Einzelheiten zu diesem Anhang : siehe Amtsblatt des Europäischen Patentamts, Nr.12/82